# EUROPEAN PATENT SPECIFICATION

(11) **EP 4 285 023 B1**
(45) Date of publication and mention of the grant of the patent: **04.11.2026**
(21) Application number: 22704229.8
(22) Date of filing: 28.01.2022
(51) Int. Cl.: F04B 9/04, F04B 13/00, F04B 19/00, F04B 23/02, A61M 5/142

(54) **HARD SEAL COMPACT, POSITIVE DISPLACEMENT PUMP WITH RECIPROCATING MOTION**
KOMPAKTE VERDRÄNGERPUMPE MIT HARTER DICHTUNG UND HIN- UND HERGEHENDER BEWEGUNG
POMPE VOLUMÉTRIQUE COMPACTE À JOINT DUR ET À MOUVEMENT ALTERNATIF

(30) Priority: 29.01.2021 US 202163143451 P
(43) Date of publication of application: 06.12.2023
(73) Proprietor: Becton, Dickinson and Company, Franklin Lakes, NJ 07417 (US)
(72) Inventor: PIZZOCHERO, Alessandro, E., Chelmsford, MA 01824 (US); WOOD, Mark, Sterling, MA 01564 (US)
(74) Representative: dompatent
(86) International application number: PCT/US2022/014228
(87) International publication number: WO 2022/165119

(56) References cited:
- WO-A1-2013/029999
- WO-A1-2015/157174
- CH-A5- 688 224
- US-A1- 2020 025 184
- US-A1- 2020 384 197

## Description

### BACKGROUND

### 1. Field

Apparatuses and methods consistent with example embodiments relate to a pump suitable for subcutaneous delivery of a liquid pharmaceutical product, and more particularly, to a hard seal, compact, positive displacement pump with a reciprocating motion.

### 2. Description of the Related Art

Diabetes is a group of diseases characterized by high levels of blood glucose resulting from the inability of diabetic patients to maintain proper levels of insulin production when required. Diabetes can be dangerous to the affected patient if it is not treated, and it can lead to serious health complications and premature death. However, such complications can be minimized by utilizing one or more treatment options to help control the diabetes and reduce the risk of complications.

The treatment options for diabetic patients include specialized diets, oral medications and/or insulin therapy. An effective method for insulin therapy and managing diabetes is infusion therapy or infusion pump therapy in which an insulin pump is used. An insulin delivery device (IDD) may include an insulin pump that can provide continuous infusion of insulin to a diabetic patient at varying rates in order to more closely match the functions and behavior of a properly operating pancreas of a non-diabetic person that produces the required insulin, and the insulin pump can help the diabetic patient maintain his/her blood glucose level within target ranges based on the diabetic patient's individual needs. Infusion pump therapy requires an infusion cannula, typically in the form of an infusion needle or a flexible catheter, that pierces the diabetic patient's skin and through which infusion of insulin takes place. Infusion pump therapy offers the advantages of continuous infusion of insulin, precision dosing, and programmable delivery schedules.

Currently, there are two principal modes of daily insulin therapy for the treatment of type 1 diabetes. The first mode includes syringes and insulin pens that require a needle stick at each injection, typically three to four times per day that are simple to use and relatively low in cost. Another widely adopted and effective method of treatment for managing diabetes is the use of an insulin pump. Insulin pumps can help the user keep blood glucose levels within target ranges based on individual needs, by continuous infusion of insulin. By using an insulin pump, the user can match insulin therapy to lifestyle, rather than matching lifestyle to how an insulin injection is working for the user.

Conventional insulin pumps are capable of delivering rapid or short-acting insulin 24 hours a day through a catheter placed under the skin. Insulin doses are typically administered at a basal rate and in a bolus dose. Basal insulin is delivered continuously over 24 hours, and keeps the user's blood glucose levels in a consistent range between meals and overnight. Some insulin pumps are capable of programming the basal rate of insulin to vary according to the different times of the day and night. Bolus doses are typically administered when the user takes a meal, and generally provide a single additional insulin injection to balance the carbohydrates consumed. Some conventional insulin pumps enable the user to program the volume of the bolus dose in accordance with the size or type of the meal consumed. Conventional insulin pumps also enable a user to take in a correctional or supplemental bolus of insulin to compensate for a low blood glucose level at the time the user is calculating a meal bolus.

There are many advantages of conventional insulin pumps over other methods of diabetes treatment. Insulin pumps deliver insulin over time rather than in single injections and thus typically result in less variation within the blood glucose range that is recommended by the American Diabetes Association. Conventional insulin pumps also reduce the number of needle sticks which the patient must endure, and make diabetes management easier and more effective for the user, thus considerably enhancing the quality of the user's life.

A major disadvantage of existing insulin pumps is that, in spite of their portability, they include multiple components and can be heavy and cumbersome to use. They are also typically more expensive than other methods of treatment. From a lifestyle standpoint, the conventional pump with its associated tubing and infusion set can be inconvenient and bothersome for the user.

Unlike a conventional infusion pump, a patch pump is an integrated device that combines most or all of the fluidic components, including the fluid reservoir, pumping mechanism and a mechanism for automatically inserting the cannula, in a single housing which is adhesively attached to an infusion site on the patient's skin, and does not require the use of a separate infusion or tubing set. Some patch pumps wirelessly communicate with a separate controller (as in one device sold by Insulet Corporation under the brand name OmniPod.RTM.), while others are completely self-contained. Such devices are replaced on a frequent basis, such as every three days, when the insulin supply is exhausted.

As a patch pump is designed to be a self-contained unit that is worn by the diabetic patient, it is preferable to be as small as possible so that it does not interfere with the activities of the user. In order to minimize discomfort to the user, it is preferable to minimize the overall dimension of the patch pump. However, in order to minimize the overall dimensions of the patch pump, its constituent parts should be reduced in size as much as possible.

Additionally, the pump, and all other portions of a patch pump or other insulin delivery device (IDD) which come into contact with the fluid or fluid path therein must be subject to sterilization. However, sterilization and ageing can drastically change the properties of elastomeric materials, and pumps utilize an elastomeric material such as liquid silicon rubber (LSR). The use of LSR in the fluid path has been shown to potentially degrade some drug formulations.
WO 2015/157174 A1 discloses a rotational metering pump for an insulin patch.

### SUMMARY

Example embodiments may address at least the above problems and/or disadvantages and other disadvantages not described above. Also, example embodiments are not required to overcome the disadvantages described above, and may not overcome any of the problems described above.

According to an aspect of an example embodiment, a positive displacement pump comprises: a housing; a sleeve disposed radially within the housing, wherein an outer conical shape of a first end of the sleeve contacts a conical inner shape of a first end of the housing, thereby sealing the first end of the sleeve to the first end of the housing; and a piston, disposed radially within the sleeve. An axially-reciprocating motion of the piston within the sleeve opens and closes a pump chamber defined between a first end of the piston and a plug disposed within the first end of the sleeve.

The pump may further comprise a cap closing a second end of the housing; and a spring, disposed between the cap and a second end of the sleeve, wherein a pressure of the spring between the cap and the housing biases the sleeve toward the first end of the housing.

The pump may further comprise a piston seal disposed at the first end of the piston, and a plug seal disposed at an end of the plug, wherein the piston seal and the plug seal define the pump chamber therebetween.

The pump may further comprise a helical slot formed in the sleeve; and a pin extending radially outward from the piston, wherein the pin is moveable within the slot, thereby controlling a movement of the piston in radial and axial directions.

The housing and the sleeve may be made of polypropylene.

The pump may further comprise an inlet port and an outlet port formed through the housing.

According to an aspect of another example embodiment, a positive displacement pump comprises: a housing; a sleeve disposed radially within the housing, wherein an outer shape of the sleeve contacts an inner shape of the housing, thereby sealing the sleeve within the housing; and a piston, disposed radially within the sleeve, wherein an wherein an axially-reciprocating motion of the piston within the sleeve opens and closes a pump chamber defined between a first end of the piston and a first end of the sleeve.

The pump may further comprise: a helical slot formed in the sleeve and the housing; and a pin extending radially outward from the piston, wherein the pin is moveable within the slot, thereby controlling a movement of the piston in radial and axial directions.

The sleeve may be rotationally moveable within the housing.

The housing and the sleeve may be made of polypropylene.

The pump may further comprise an inlet port and an outlet port formed through the housing.

According to an aspect of another example embodiment, a fluid delivery system comprises: a reservoir; a cannula; and a pump according to one of the example embodiments described above. The inlet port of the pump is in fluid communication with the reservoir and the outlet port of the pump is in fluid communication with the cannula.

### BRIEF DESCRIPTION OF THE DRAWINGS

The above and/or other example aspects and advantages will become apparent and more readily appreciated from the following description of example embodiments, taken in conjunction with the accompanying drawings in which:
FIG. 1 is a schematic overview of a fluid delivery system according to an example embodiment;
FIG. 2A is a perspective, cutaway view of a pump, according to a first example embodiment;
FIG. 2B is another perspective, cutaway view of the pump according to the first example embodiment;
FIG. 2C is another perspective, cutaway view of the pump according to the first example embodiment;
FIG. 3 is a perspective view of a piston, seal, and plug of the pump according to the first example embodiment;
FIG. 4A is a perspective, cutaway view of a pump, according to a second example embodiment;
FIG. 4B is another perspective, cutaway view of the pump according to the second example embodiment; and
FIG. 4C is a perspective view of the pump according to the second example embodiment.

### DETAILED DESCRIPTION

Reference will now be made in detail to example embodiments which are illustrated in the accompanying drawings, wherein like reference numerals refer to like elements throughout. In this regard, the example embodiments may have different forms and may not be construed as being limited to the descriptions set forth herein.

It will be understood that the terms "include," "including," "comprise," and/or "comprising," when used in this specification, specify the presence of stated features, integers, steps, operations, elements, and/or components, but do not preclude the presence or addition of one or more other features, integers, steps, operations, elements, components, and/or groups thereof

It will be further understood that, although the terms "first," "second," "third," etc., may be used herein to describe various elements, components, regions, layers and/or sections, these elements, components, regions, layers and/or sections may not be limited by these terms. These terms are only used to distinguish one element, component, region, layer or section from another element, component, region, layer or section.

As used herein, the term "and/or" includes any and all combinations of one or more of the associated listed items. Expressions such as "at least one of," when preceding a list of elements, modify the entire list of elements and do not modify the individual elements of the list. In addition, the terms such as "unit," "-er (-or)," and "module" described in the specification refer to an element for performing at least one function or operation, and may be implemented in hardware, software, or the combination of hardware and software.

Various terms are used to refer to particular system components. Different companies may refer to a component by different names - this document does not intend to distinguish between components that differ in name but not function.

Matters of these example embodiments that are obvious to those of ordinary skill in the technical field to which these example embodiments pertain may not be described here in detail.

One or more example embodiments describe may utilize a hard seal which removes the potentially destabilizing elastomeric material, such as LSR, from the fluid path. An interlock may also be omitted from the pump in order to make the pump smaller, with a lower part count, thereby making it easier to assemble and install. One or more example embodiments may also Improve the fit of a drive cross-pin in the piston and resize related components to avoid dosing errors. The helix may be mirrored/reversed in order that the cross-pin may contact on two, opposite sides, balancing loads and kinematic motion, leading to an improved dose accuracy and more stable operation.

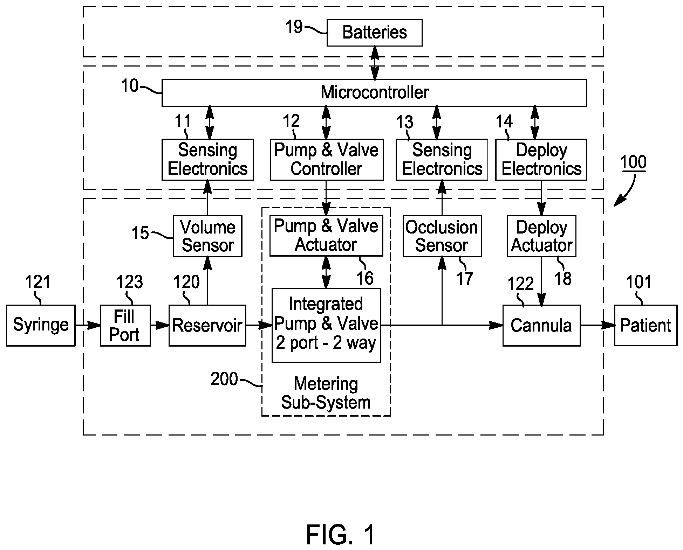

FIG. 1 is a schematic overview of a fluid delivery system 100, comprising a reservoir 120 in fluid communication with a metering subsystem (the pump) 200 for drawing a precise amount of fluid from the reservoir, and a cannula mechanism 122 for delivering medication to the user 101. The cannula mechanism 122 may be connected to the infusion site by an infusion set comprising tubing and a patch, or alternatively a cannula insertion mechanism maybe incorporated into a housing within the metering subsystem 200. Although example embodiments are not limited to any specific reservoir configuration, the reservoir 120 may be flexible. A flexible reservoir does not have an internal actuator mechanism for delivering fluid, which permits the overall pump 200 to have a smaller footprint and more compact design. The reservoir may be filled via a fill port 123 by syringe 121, for example, or a prefilled reservoir or cartridge may be used.

A microcontroller 10 may take the form of a printed circuit board (PCB) which interfaces with sensors and circuitry 11, 12, 13, 14, 15, 17 and with actuators 16 and 18, to control the pump and cannula. Power is provided by one or more batteries 19 in the housing. Audible feedback and visual display and user operable controls (not shown) may be provided on the unit, operatively connected to the PCB, or on a remote programming unit, to set dosage, deploy the cannula, initiate infusion and deliver bolus dosages.

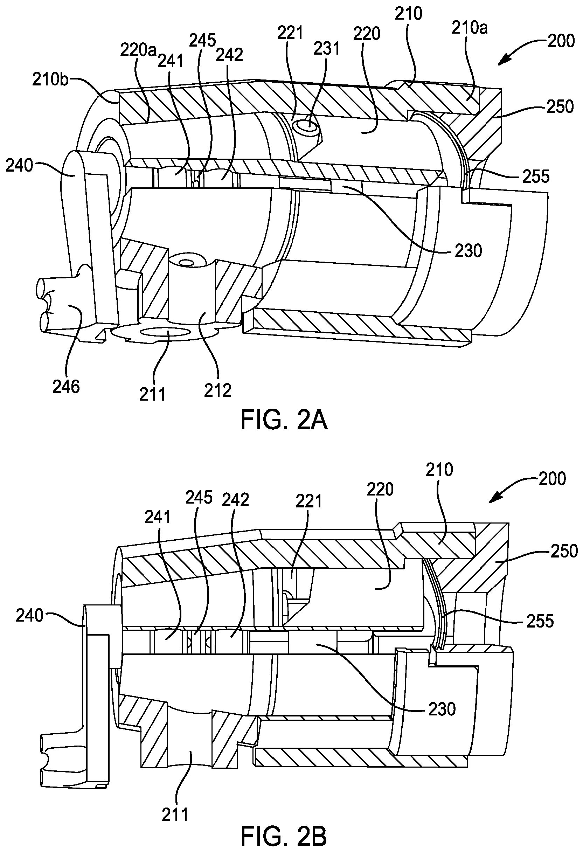

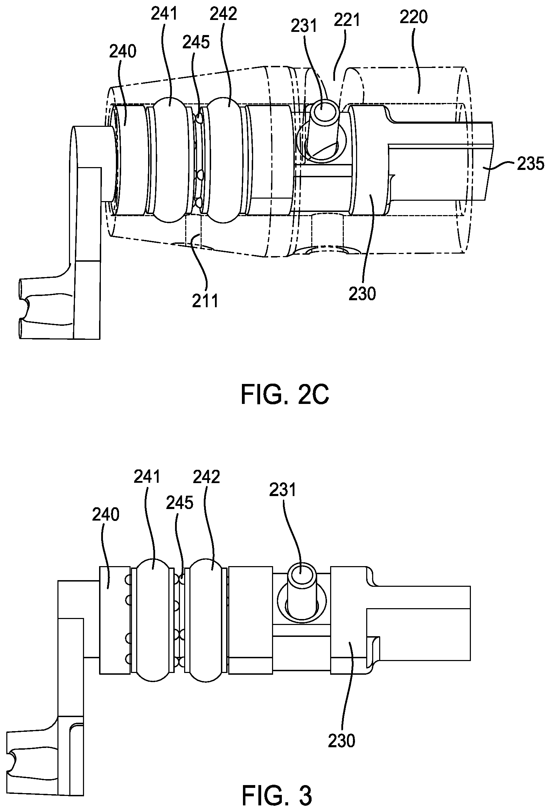

FIGs. 2A, 2B, and 2C illustrate a pump 200 according to a first example embodiment. FIG. 2A is a perspective view of the pump 200, including a housing 210; a load cap 250, closing one end of the housing 210; a sleeve 220 disposed within the housing; and a plug 240, a sealing portion, and a piston 230, disposed within the sleeve 220. The housing 210 has one end 210a closed by the load cap 250, and a second end 210b closed by the sleeve 220 and the plug 240. A wave washer spring 255 is disposed between the cap 250 and one end of the sleeve 220, and the other end of the sleeve 220 comprises an outer conical shape which fits within a corresponding inner conical shape of the housing 210 at a conical interface 220a. In this way, the cap 250 presses on the spring 255, thereby holding the conical shape of the sleeve 220 within the conical shape of the housing 210. This means of holding the sleeve within the housing is merely an example. The sleeve and housing may have shapes other than conical, and the sleeve may be pressed and held within the housing by another means, such as heat-staking, laser welding, bonding, or another means, as would be understood in order to create a sealing force between the sleeve 220 and the housing 210. Additionally, the spring 255, described as a wave washer, may alternately be another type of spring, or an elastomer material which provides the force. A lubricant may be used to control friction and wear characteristics among the various components of the pump 200.

The housing has an inlet port 211 therein, in fluid communication with a fluid path from the reservoir 120 to the pump 200, and an outlet port 212 therein, in fluid communication with a fluid path from the pump 200 to the cannula 122. Within the pump 200, the inlet port 211 and outlet port 212 may communicate with a pump chamber 245 inside the sleeve 220, based on a position of the piston 230. The ports 211, 212 may be chamfered to improve an alignment overlap, and one or more switches (not shown) may be placed onto the pump 200 to detect limits of motion to reverse the motor rotation. Within the sleeve, the pump chamber 245 is bounded by a plug seal 241, on a side of the plug 240, and a piston seal 242, on a side of the piston 230. The plug 240 itself may be glued to the sleeve 220 in assembly and rotates with the sleeve 220.

A cross-pin 231 extends radially outward from the piston 230, and moves within a helical slot 221 in the sleeve 220. The sleeve 220 is fixed within the housing 210 both rotationally and axially. A rotation of the piston 230 moves the pin 231 within the slot 221, which is formed helically around the sleeve. With respect to this example aspect, the slot 221 is helical. However, as discussed above, the sleeve and housing may be other than conical, and accordingly, the slot may be other than helical, as would be understood by one of skill in the art. Thus, as the piston 230 rotates, the pin 231 moves within the slot 221, causing the piston 230 to also move towards and away from the plug 240, moving the piston seal 242, and opening and closing the pump chamber 245. The piston 230 may have a flat tab 235 on one end, as shown in FIG. 2C, with o-rings thereon, such that one o-ring moves with the piston 230 and one o-ring moves with the sleeve 220

The plug 240 may include a handle 246 which rotates and moves with the pin 231, in order to trigger a switch (not shown) which detects the angular position of the plug 240.

According to this example embodiment, the components of the sleeve 220 and the housing 210 are formed of hard plastic and are held together by pressure sufficient to hold during rotation and after sterilization and aging. The hard plastic may be Vespel or polypropylene, as would be understood by one of skill in the art.

The pump 200 may be driven by a stepper motor (not shown) between a first angular position and a second angular position, respectively representing the two extreme positions of the piston in normal operation. When the pump 220 moves from a first position to an open position, the pump chamber 245 opens, and is in communication with the inlet port 211, pulling fluid from the reservoir 120 into the pump chamber 245. When the pump 200 moves from the open position to a second position, the pump chamber 245 closes, and is in communication with the outlet port 212, pumping the fluid into the outlet port 212 toward the cannula 122.

FIG. 3 is a perspective view of the piston, plug, and seal portions of the interior of the pump 200, according to the first example embodiment.

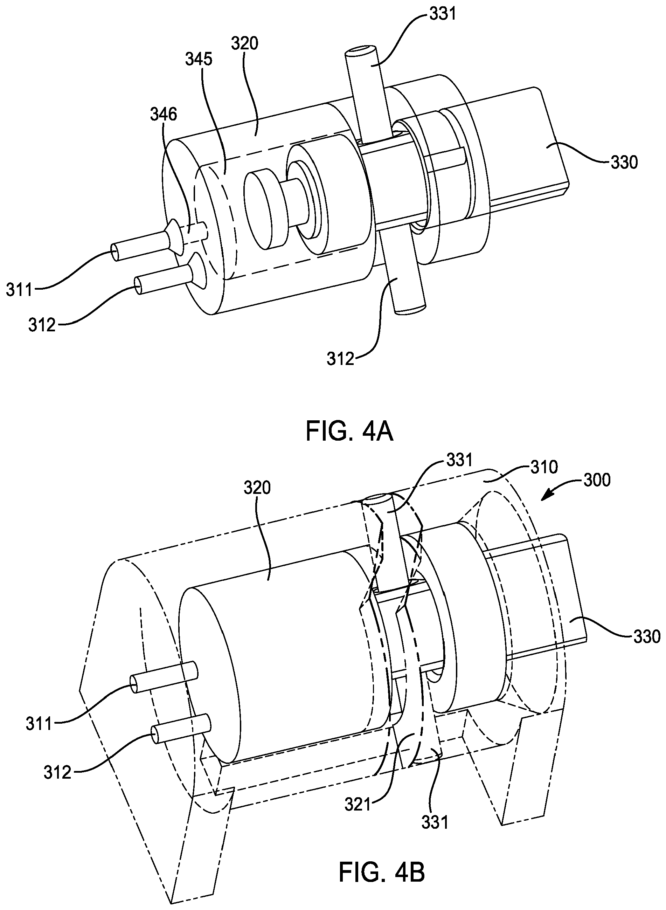

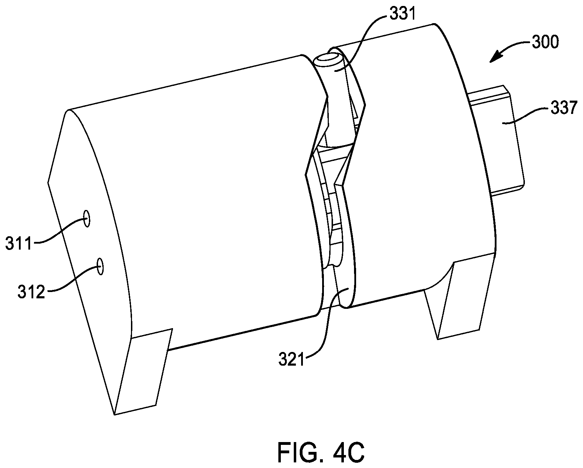

FIGs. 4A, 4B, and 4C illustrate a pump 300 according to a second example embodiment. FIG. 4A is a perspective view of a portion of the pump including a sleeve 320 and a piston 330, disposed within the sleeve 320. A housing 310, surrounding the sleeve 320, is shown in FIGs. 4B and 4C. The housing 310 has one end from which the piston 330 protrudes, and a second end having formed therein an inlet port 311 and an outlet port 312. The sleeve 320 is disposed within the housing 310, and the piston 330 moves longitudinally with respect to the sleeve 320, while the sleeve 320 may rotate within the housing 310. The pump chamber 345 is defined between an end of the piston 330 and an end of the sleeve 320, wherein the end of the sleeve 320 has a sleeve port 346 formed therethrough. The pump chamber 345 within the sleeve 320 may thereby be in communication with the inlet port 311 or the outlet port 312, via the sleeve port, depending on the rotation of the sleeve 320.

A dual cross-pin 331 extends radially outward, in opposite directions, from the piston 330, and moves within a slot 321 in the sleeve 320 and the housing 310, as shown in FIGs. 4B and 4C. The sleeve 320 is axially fixed within the housing 310, but may rotate within the housing 310, thereby bringing either the inlet port 311 or the outlet port 312 into communication with the pump chamber 345, via the sleeve port 346.

The piston 330 may rotate and move axially within the sleeve 320. A rotation of the piston 330 moves the pin 331 within the slot 321 in the sleeve 320 and the housing 310. In an inlet closed position, the piston is pressed against the end of the housing 310, closing the pump chamber 345, and the sleeve 320 is rotated such that the sleeve port 346 is in communication with the inlet port 311. As the piston 330 moves from the inlet closed position to an inlet open position, the piston is pulled away from the pump chamber 345, opening the pump chamber 345, and pulling fluid into the pump chamber 345 from the reservoir 120. The sleeve 320 is then rotated from a position in which the sleeve port 346 is in communication with the inlet port 311 to a position in which the sleeve port 346 is in communication with the outlet port 312. The piston 330 then moves from an outlet open position to an outlet closed position, the rotation of the piston 330 moving the piston to close the pump chamber 345, pumping fluid from the pump chamber 345 to the cannula 122. When the piston 330 is in the closed position, the sleeve 320 is then switched again from a position in which the sleeve port 346 is in communication with the outlet port 312 to a position in which the sleeve port 346 is in communication with the inlet port 311.

According to this example embodiment, the components of the sleeve 320 and the housing 310 are formed of hard plastic and are held together by pressure sufficient to hold during rotation and after sterilization and aging.

As with the first example embodiment, the pump 300 may be driven by a stepper motor (not shown).

It may be understood that the example embodiments described herein may be considered in a descriptive sense only and not for purposes of limitation. Descriptions of features or aspects within each example embodiment may be considered as available for other similar features or aspects in other example embodiments.

While example embodiments have been described with reference to the figures, it will be understood by those of ordinary skill in the art that various changes in form and details may be made therein without departing from the scope as defined by the following claims.

## Claims

1. A positive displacement pump comprising:
a housing (210) comprising a first end (210a) and a second end (210b);
an inlet port (211);
an outlet port (212);
a sleeve (220) disposed within the housing (210), the sleeve comprising a proximal end and a distal end opposite the proximal end;
a plug (240) disposed within the sleeve (220), and sealing the proximal end of the sleeve (220);
a pump chamber (245) inside the sleeve (220); and
a piston (230) disposed within the sleeve (220), wherein an axially-reciprocating motion of the piston (230) within the sleeve (220) opens and closes the pump chamber (245) whereby the inlet port (211) and outlet port (212) may communicate with the pump chamber (245) inside the sleeve (220) based on a position of the piston (230),
**characterized in that**
an outer shape of the proximal end of the sleeve (220) contacts an inner shape of the second end (210b) of the housing (210), wherein the inner shape of the second end (210b) of the housing (210) corresponds to the outer shape of the proximal end of the sleeve (220); and
a means of pressing and holding the sleeve (220) within the housing (210) creates a sealing force between the sleeve (220) and the housing (210), and a spring (255) or elastomer material provides said sealing force thereby sealing the proximal end of the sleeve (220) to the second end (210b) of the housing (210).

2. The positive displacement pump according to claim 1, wherein the outer shape of the proximal end of the sleeve (220) and the inner shape of the second end (210b) of the housing (210) are conical.

3. The positive displacement pump according to claim 1 or 2, further comprising a cap (250) closing the first end (210a) of the housing (210).

4. The positive displacement pump according to claim 3, wherein the means of holding the sleeve (220) within the housing (210) presses the sleeve (220) to hold the outer shape of the proximal end of the sleeve (220) in contact with the inner shape of the second end (210b) of the housing (210).

5. The positive displacement pump according to claim 4, wherein the means of pressing the sleeve (220) comprises a means of biasing the sleeve (220) toward the second end (210b) of the housing (210).

6. The positive displacement pump according to claim 5, wherein the means of biasing the sleeve (220) comprises a spring (255).

7. The positive displacement pump according to claim 6, wherein
the spring (255) is disposed between the cap (250) and the distal end of the sleeve (220), and
a pressure of the spring (255) between the cap (250) and the sleeve (220) biases the sleeve (220) toward the second end (210b) of the housing (210).

8. The positive displacement pump according to one of claims 1 - 7, further comprising:
a piston seal (242) disposed on a side of the piston (230), and
a plug seal (241) disposed on a side of the plug (240),
wherein the pump chamber (245) is bounded by the piston seal (242) and the plug seal (241).

9. The positive displacement pump according to one of claims 1 - 8, further comprising:
a slot (221) formed in the sleeve (220); and
a pin (231) extending radially outward from the piston (230),
wherein, as the piston (230) rotates, the pin (231) moves within the slot (221), causing the piston (230) to move in axial directions opening and closing the pump chamber (245).

10. The positive displacement pump according to claim 9, wherein the slot (221) is helical.

11. The positive displacement pump according to one of claims 1 - 10, wherein the housing (210) and the sleeve (220) are formed of hard plastic and are held together by a pressure sufficient to hold during a rotation of the piston (230) within the sleeve (220).

12. The positive displacement pump according to one of claims 1 - 11, wherein the housing and the sleeve are made of polypropylene.

13. The positive displacement pump according to one of claims 1 - 12, wherein the inlet port (211) and the outlet port (212) are formed through the housing (210).

14. A fluid delivery system comprising the pump (200) according to claim 13, the fluid delivery system further comprising:
a reservoir (120); and
a cannula (122) ,
wherein the inlet port (211) is in fluid communication with the reservoir (120) and the outlet port (212) is in fluid communication with the cannula (122); and
wherein the piston (230) is moveable between an inlet position, in which the pump chamber (245) is in communication with the inlet port (211), pulling fluid from the reservoir 120 into the pump chamber (245), and an outlet position, in which the pump chamber (245) is in communication with the outlet port (212), pumping the fluid into the outlet port (212) toward the cannula (122).

15. The fluid delivery system of claim 14 further comprising
a stepper motor driving the piston (230) between a first angular position and a second angular position, respectively, causing the piston (230) to move in axial directions between the inlet position and the outlet position.

## Patentansprüche

1. Verdrängerpumpe, die aufweist:
ein Gehäuse (210), das ein erstes Ende (210a) und ein zweites Ende (210b) aufweist;
eine Einlassöffnung (211);
eine Auslassöffnung (212);
eine Hülse (220), die innerhalb des Gehäuses (210) angeordnet ist, wobei die Hülse ein proximales Ende und ein dem proximalen Ende entgegengesetztes distales Ende aufweist;
ein Stopfen (240), der innerhalb der Hülse (220) angeordnet ist und das proximale Ende der Hülse (220) abdichtet;
eine Pumpenkammer (245) innerhalb der Hülse (220); und
ein Kolben (230), der innerhalb der Hülse (220) angeordnet ist, wobei eine axiale Hin- und Herbewegung des Kolbens (230) innerhalb der Hülse (220) die Pumpenkammer (245) öffnet und schließt, wodurch die Einlassöffnung (211) und die Auslassöffnung (212) je nach Position des Kolbens (230) mit der Pumpenkammer (245) im Inneren der Hülse (220) in Verbindung treten können,
**dadurch gekennzeichnet, dass**
eine äußere Form des proximalen Endes der Hülse (220) eine innere Form des zweiten Endes (210b) des Gehäuses (210) berührt, wobei die innere Form des zweiten Endes (210b) des Gehäuses (210) der äußeren Form des proximalen Endes der Hülse (220) entspricht; und
ein Mittel zum Drücken und Festhalten der Hülse (220) innerhalb des Gehäuses (210) eine Dichtungskraft zwischen der Hülse (220) und dem Gehäuse (210) erzeugt, und eine Feder (255) oder ein Elastomermaterial für diese Dichtkraft sorgt, wodurch das proximale Ende der Hülse (220) am zweiten Ende (210b) des Gehäuses (210) abgedichtet wird.

2. Verdrängerpumpe nach Anspruch 1, wobei die äußere Form des proximalen Endes der Hülse (220) und die innere Form des zweiten Endes (210b) des Gehäuses (210) konisch sind.

3. Verdrängerpumpe nach Anspruch 1 oder 2, die ferner eine Kappe (250) aufweist, die das erste Ende (210a) des Gehäuses (210) verschließt.

4. Verdrängerpumpe nach Anspruch 3, wobei das Mittel zum Halten der Hülse (220) innerhalb des Gehäuses (210) die Hülse (220) drückt, um die äußere Form des proximalen Endes der Hülse (220) in Kontakt mit der inneren Form des zweiten Endes (210b) des Gehäuses (210) zu halten.

5. Verdrängerpumpe nach Anspruch 4, wobei das Mittel zum Drücken der Hülse (220) ein Mittel zum Vorspannen der Hülse (220) in Richtung des zweiten Endes (210b) des Gehäuses (210) aufweist.

6. Verdrängerpumpe nach Anspruch 5, wobei das Mittel zum Vorspannen der Hülse (220) eine Feder (255) aufweist.

7. Verdrängerpumpe nach Anspruch 6, wobei
die Feder (255) zwischen der Kappe (250) und dem distalen Ende der Hülse (220) angeordnet ist, und
durch den Druck der Feder (255) zwischen der Kappe (250) und der Hülse (220) die Hülse (220) in Richtung des zweiten Endes (210b) des Gehäuses (210) vorgespannt wird.

8. Verdrängerpumpe nach einem der Ansprüche 1-7, die ferner aufweist:
eine Kolbendichtung (242), die an einer Seite des Kolbens (230) angeordnet ist, und
eine Kolbendichtung (241), die an einer Seite des Stopfens (240) angeordnet ist, und
wobei die Pumpenkammer (245) durch die Kolbendichtung (242) und die Stopfendichtung (241) begrenzt wird.

9. Verdrängerpumpe nach einem der Ansprüche 1-8, die ferner aufweist:
einen Spalt (221), der in der Hülse (220) ausgebildet ist; und
ein Stift (231), der sich vom Kolben (230) radial nach außen erstreckt,
wobei sich der Stift (231) bei der Drehung des Kolbens (230) innerhalb des Spalts (221) bewegt, wodurch sich der Kolben (230) in axiale Richtungen bewegt und die Pumpenkammer (245) öffnet und schließt.

10. Verdrängerpumpe nach Anspruch 9, wobei der Spalt (221) schraubenförmig ist.

11. Verdrängerpumpe nach einem der Ansprüche 1-10, wobei das Gehäuse (210) und die Hülse (220) aus Hartkunststoff ausgebildet sind und durch einen Druck zusammengehalten werden, der ausreicht, um sie während einer Drehung des Kolbens (230) innerhalb der Hülse (220) zu halten.

12. Verdrängerpumpe nach einem der Ansprüche 1-11, wobei das Gehäuse und die Hülse aus Polypropylen bestehen.

13. Verdrängerpumpe nach einem der Ansprüche 1-12, wobei die Einlassöffnung (211) und die Auslassöffnung (212) durch das Gehäuse (210) hindurch ausgebildet sind.

14. Fluidabgabesystem, das die Pumpe (200) nach Anspruch 13 aufweist, wobei das Fluidabgabesystem ferner aufweist:
ein Reservoir (120); und
eine Kanüle (122),
wobei die Einlassöffnung (211) in Fluidverbindung mit dem Reservoir (120) steht und die Auslassöffnung (212) in Fluidverbindung mit der Kanüle (122) steht; und
wobei der Kolben (230) zwischen einer Einlassposition, in der die Pumpenkammer (245) mit der Einlassöffnung (211) in Verbindung steht und Fluid aus dem Reservoir (120) in die Pumpenkammer (245) saugt, und einer Auslassposition, in der die Pumpenkammer (245) mit der Auslassöffnung (212) in Verbindung steht und das Fluid in die Auslassöffnung (212) in Richtung der Kanüle (122) pumpt, bewegbar ist.

15. Fluidabgabesystem nach Anspruch 14, das ferner aufweist:
einen Schrittmotor, der den Kolben (230) zwischen einer ersten Winkelposition und einer zweiten Winkelposition antreibt und dadurch bewirkt, dass sich der Kolben (230) in axialen Richtungen zwischen der Einlassposition und der Auslassposition bewegt.

## Revendications

1. Pompe volumétrique comprenant :
un carter (210) comprenant une première extrémité (210a) et une seconde extrémité (210b) ;
un orifice d'entrée (211) ;
un orifice de sortie (212) ;
un manchon (220) disposé à l'intérieur du carter (210), le manchon comprenant une extrémité proximale et une extrémité distale opposée à l'extrémité proximale ;
un bouchon (240) disposé à l'intérieur du manchon (220), et scellant l'extrémité proximale du manchon (220) ;
une chambre de pompe (245) à l'intérieur du manchon (220) ; et
un piston (230) disposé à l'intérieur du manchon (220), dans laquelle un mouvement de va-et-vient axial du piston (230) à l'intérieur du manchon (220) ouvre et ferme la chambre de pompe (245) de sorte que l'orifice d'entrée (211) et l'orifice de sortie (212) peuvent communiquer avec la chambre de pompe (245) à l'intérieur du manchon (220) en fonction d'une position du piston (230)
**caractérisée en ce que**
une forme extérieure de l'extrémité proximale du manchon (220) entre en contact avec une forme intérieure de la seconde extrémité (210b) du carter (210), dans laquelle la forme intérieure de la seconde extrémité (210b) du carter (210) correspond à la forme extérieure de l'extrémité proximale du manchon (220) ; et
un moyen de pressage et de maintien du manchon (220) à l'intérieur du carter (210) crée une force d'étanchéité entre le manchon (220) et le carter (210), et un ressort (255) ou matériau élastomère fournit ladite force d'étanchéité, scellant ainsi l'extrémité proximale du manchon (220) à la seconde extrémité (210b) du carter (210).

2. Pompe volumétrique selon la revendication 1, dans laquelle la forme extérieure de l'extrémité proximale du manchon (220) et la forme intérieure de la seconde extrémité (210b) du carter (210) sont coniques.

3. Pompe volumétrique selon la revendication 1 ou 2, comprenant en outre un capuchon (250) fermant la première extrémité (210a) du carter (210).

4. Pompe volumétrique selon la revendication 3, dans laquelle le moyen de maintien du manchon (220) à l'intérieur du carter (210) presse le manchon (220) pour maintenir la forme extérieure de l'extrémité proximale du manchon (220) en contact avec la forme intérieure de la seconde extrémité (210b) du carter (210).

5. Pompe volumétrique selon la revendication 4, dans laquelle le moyen de pressage du manchon (220) comprend un moyen de sollicitation du manchon (220) vers la seconde extrémité (210b) du carter (210).

6. Pompe volumétrique selon la revendication 5, dans laquelle le moyen de sollicitation du manchon (220) comprend un ressort (255).

7. Pompe volumétrique selon la revendication 6, dans laquelle
le ressort (255) est disposé entre le capuchon (250) et l'extrémité distale du manchon (220), et
une pression du ressort (255) entre le capuchon (250) et le manchon (220) sollicite le manchon (220) en direction de la seconde extrémité (210b) du carter (210).

8. Pompe volumétrique selon l'une des revendications 1 - 7, comprenant en outre :
un joint de piston (242) disposé sur un côté du piston (230), et
un joint de bouchon (241) disposé sur un côté du bouchon (240),
dans laquelle la chambre de pompe (245) est délimitée par le joint de piston (242) et le joint de bouchon (241).

9. Pompe volumétrique selon l'une des revendications 1 - 8, comprenant en outre :
une fente (221) formée dans le manchon (220) ; et
une goupille (231) s'étendant radialement vers l'extérieur depuis le piston (230),
dans laquelle, à mesure que le piston (230) tourne, la goupille (231) se déplace dans la fente (221), amenant le piston (230) à se déplacer dans des directions axiales ouvrant et fermant la chambre de pompe (245).

10. Pompe volumétrique selon la revendication 9, dans laquelle la fente (221) est hélicoïdale.

11. Pompe volumétrique selon l'une des revendications 1 - 10, dans laquelle le carter (210) et le manchon (220) sont formés en plastique dur et sont maintenus ensemble par une pression suffisante pour assurer le maintien pendant une rotation du piston (230) à l'intérieur du manchon (220).

12. Pompe volumétrique selon l'une des revendications 1 - 11, dans laquelle le carter et le manchon sont en polypropylène.

13. Pompe volumétrique selon l'une des revendications 1 - 12, dans laquelle l'orifice d'entrée (211) et l'orifice de sortie (212) sont formés à travers le carter (210).

14. Système de distribution de fluide comprenant la pompe (200) selon la revendication 13, le système de distribution de fluide comprenant en outre :
un réservoir (120) ; et
une canule (122),
dans lequel l'orifice d'entrée (211) est en communication fluidique avec le réservoir (120) et l'orifice de sortie (212) est en communication fluidique avec la canule (122) ; et
dans lequel le piston (230) est mobile entre une position d'entrée, dans laquelle la chambre de pompe (245) est en communication avec l'orifice d'entrée (211), aspirant le fluide du réservoir 120 dans la chambre de pompe (245), et une position de sortie, dans laquelle la chambre de pompe (245) est en communication avec l'orifice de sortie (212), pompant le fluide dans l'orifice de sortie (212) en direction de la canule (122).

15. Système de distribution de fluide selon la revendication 14 comprenant en outre
un moteur pas à pas entraînant le piston (230) entre une première position angulaire et une seconde position angulaire, respectivement, amenant le piston (230) à se déplacer dans des directions axiales entre la position d'entrée et la position de sortie.
